# Europäisches Patentamt

## European Patent Office

## Office européen des brevets

(11) Publication number: **0 106 727**
**B1**

(12) # EUROPEAN PATENT SPECIFICATION

(45) Date of publication of patent specification: **16.08.89**

(51) Int. Cl.⁴: **A 61 K 47/00** // C07C101/04

(21) Application number: **83401762.6**

(22) Date of filing: **07.09.83**

(54) **Method of improving parenterally administerable physiologically active compositions by incorporating therein an epsilon-aminocaproic acid or tranexamic acid.**

(30) Priority: **07.09.82 US 415567**
**07.09.82 US 415568**
**07.09.82 US 415569**

(43) Date of publication of application:
**25.04.84 Bulletin 84/17**

(45) Publication of the grant of the patent:
**16.08.89 Bulletin 89/33**

(84) Designated Contracting States:
**AT BE CH DE FR GB IT LI LU NL SE**

(56) References cited:
**LU-A- 34 976**
**LU-A- 58 953**

The file contains technical information
submitted after the application was filed and
not included in this specification

(73) Proprietor: **Tetra Consultants Inc.**
**P.O.Box 66 Wykagyl Station**
**New Rochelle, N.Y. 10804 (US)**

(72) Inventor: **Stevens, Eric**
**Heriosanlaan 44**
**Linden-Lubbeck (BE)**
Inventor: **Van Hoeyveld, Ernestina Maria**
**Vosken 3A**
**Lubbeck (BE)**

(74) Representative: **Maiffret, Bernard**
**Law Offices of William J. Rezac 49, avenue**
**Franklin D. Roosevelt**
**F-75008 Paris (FR)**

(56) References cited:
**CHEMICAL ABSTRACTS, vol. 96, no. 2, 11th**
**January 1982, page 329, no. 11604h, Columbus,**
**Ohio, US; I.A. CHAUDRY et al.: "Compatibility**
**of netilmicin sulfate injection with commonly**
**used intravenous injections and additives" &**
**AM. J. HOSP. PHARM. 1981, 38(11), 1737-1742**

References cited:

CHEMICAL ABSTRACTS, vol. 98, no. 26, 27th
June 1983, page 372, no. 221706k, Columbus,
Ohio, US; A. KOSHIRO et al.: "Compatibility of
gentamicin sulfate injection in saline solution.
II." & YAKUZAIGAKU 1982, 42(4), 365-369

CHEMICAL ABSTRACTS, vol. 98, no. 26, 27th
June 1983, page 372, no. 221707m, Columbus,
Ohio, US; A. KOSHIRO et al.: "Stability of
cefotiam in the parenteral admixture" &
YAKUZAIGAKU 1982, 42(4), 370-376

DERWENT JAPANESE PATENTS REPORT, vol.
4, no. 45, published 10th December 1965; & JP -
B - 65 25632 (CHUGAI PHARM. CO. LTD.)

## Description

This invention relates to methods of producing parenterally administerable physiologically active compositions containing allergen extracts and improved compositions useful for such purposes. More particularly, this invention has as its objective improved and novel methods for parenterally administering liquid, physiologically active compositions to patients and to new compositions useful therefor as defined below.

It is well recognized that there are numerous instances in which physiologically active compositions are required to be parenterally administered to patients. Physiologically active compositions may have to be parenterally administered to patients for either therapeutic or diagnostic purposes and physiologically active components of the compositions can encompass a broad spectrum of active materials including such well known materials as biological derivatives, for example, sera, vaccines, toxoids and anti-toxins, allergens, and allergenic extracts; or chemically derived substances, for example, antibiotics, steroids, central nervous system agents, and other pharmacologically active chemical substances.

When it is determined by the skilled worker that the physiologically active material to be administered is best introduced to the patient being treated by a parenteral route, the physiologically active material is first incorporated into a suitable parenterally administerable composition for this purpose. This parenterally administerable composition is usually in a liquid form and is usually comprised of a suitable, parenterally administerable non-toxic vehicle, for example, water or a combination of water and alcohol, into which has been incorporated the required amount of the desired physiologically active material. The resultant liquid parenterally administerable physiologically active composition is then administered to the patient by the parenteral route selected by the skilled worker, for example, by intramuscular, subcutaneous, intradermal or intravenous route, employing the usual acceptable means for accomplishing this administration, such as a hypodermic needle and syringe. The methods and means of parenterally administering such parenterally administerable compositions to patients are well known to and understood by the worker skilled in the art.

On occasion, as a result of the parenteral administration of these physiologically active compositions, the patient suffers various adverse systemic side effects, for example, swelling and inflammation of the skin and subdermal area surrounding the site of parenteral administration. While these localized systemic adverse reactions are usually not severe or frequent, they do occur with sufficient frequency and intensity in the parenteral administration of certain physiologically active materials, for example, allergenic extracts, insect or snake venoms, certain insoluble antibiotics, to pose a serious problem to the patient being treated. Likewise, the incidence and severity of these adverse reactions may also increase in response to the character of the parenterally administerable vehicle being employed in the final compositions being administered to the patient; for example, vegetable oil vehicles frequently cause such adverse reactions. To date, though many various attempts have been made to reduce or eliminate the occurrence of these adverse reactions, none has been completely satisfactory.

It has historically been widely recognized that various physiologically active compositions which are designed for parenteral administration to patients are susceptible to rapid deterioration. This is especially true in the case of those physiologically active compositions which are in the form of a liquid parenterally administerable composition. The deterioration of these parenterally administerable physiological compositions results in loss of potency or degradation of the physiologically active components thereof. This storage instability and the resulting degradation of physiologically active components of these compositions is most prevalent in those parenterally administerable compositions which employ an aqueous vehicle.

In the past, this serious disadvantage of the degradation and storage instability of the liquid parenterally administerable physiologically active compositions has been sought to be overcome by various means, none of which have proven to be completely satisfactory.

Heretofore, many attempts have been made to solve this instability problem, most of which involve the incorporation into the parenterally administerable composition of substantial amounts of various agents which were supposed to obviate this problem. Among the various agents which have been employed for this purpose may be included such materials as glycerin; polysorbate® 80 (polyoxyethylene (20) sorbitan monooleate); dextrose; human serum albumin; aluminum precipitation; siliconization of the vials used to store the compositions; buffered saline solutions, for example, phosphate- or bicarbonate-buffered; and various other materials, for example, polyvinyl pyrrolidone or polyethylene glycol. To date none of the foregoing has provided the satisfactory results required.

Derwent Japanese Patent Report, Vol. 4, No. 45, December 10, 1965, and JP—B—6 525 643 disclose the stabilization of pyridoxal-5′-phosphate solution by adding 0,5—4 mols of potassium metabisulfite and 1—2 mols of epsilon-aminocaproic acid to the solution based on 1 mol of pyridoxal-5′-phosphate. There is no indication in this reference as to what kind of stability is imparted by the disclosed stabilization system.

LU—A—34976 discloses a process for dissolving thyrotricin and other antibiotics of peptid character, which are either insoluble or hardly soluble in water, as well as candicidin, in water and liquid media, and for improving the solubility of said antibiotics. According to claim 1, aliphatic amines having one or more amino groups, or salts thereof, particularly those having a linear chain of from 6 to 18 carbon atoms, are used as solubilizing means. In all the examples, the aliphatic amines have at least one linear chain of at least 12 carbon atoms.

LU—A—58 953 discloses a process for preparing an improved diagnostic and therapeutic allergenic extract from a substance, comprising the steps of mixing the substance with an extracting fluid comprising water and organic solvent containing basic nitrogen, separating the extracting fluid from all the insoluble matters contained therein, removing the insoluble matters, and then evaporating the extracting fluid to isolate the active principles from said substance in the form of a stable, dry, active amine such as pyridine, quinoline, lutidine, cellidine and picoline.

We have now discovered that the disadvantages suffered by the prior art parenterally administerable physiologically active compositions may be overcome by the practice of the instant invention. We have found that liquid, parenterally administerable, physiologically active compositions may be vastly improved by the incorporation therein of a small but effective amount of *epsilon*-aminocaproic acid or a non-toxic pharmaceutically acceptable salt thereof (EACA).

EACA has been found to provide satisfactory results when incorporated into the subject liquid parenterally administerable, physiologically active compositions in small but effective amounts. We have found that the amounts of EACA which may be employed herein should be sufficient to provide a final concentration thereof in the liquid, parenterally administerable, physiologically active composition to be administered to the patient, of from about 0.001 M to about 0.5 M. Preferably, in the practice of this invention EACA may be present in the final, liquid parenterally administerable, physiologically active compositions of this invention in a concentration of from about 0.01 M to about 0.5 M, and most preferably in a concentration of from about 0.1 M to about 0.25 M, although the other concentrations also provide satisfactory results.

The parenterally administerable, physiologically active compositions which may be employed in the practice of this invention include those parenterally administerable compositions which are stored and/or administered to the patient being treated in liquid form, and which are subject to deterioration, degradation or loss of potency on standing. More particularly, the compositions which may be employed in the practice of this invention are those which are in liquid form, for example, solutions, suspensions or mixtures, where the liquid vehicle is one which is a pharmaceutically acceptable liquid vehicle for parenteral administration, for example, water, oil or alcohol, and which contains a physiologically active agent which is to be parenterally administered to the patient being treated. Parenteral administration of the final compositions of this invention may be accomplished by the administration thereof by any of the following routes: subcataneous, intradermal, intramuscular, intravenous or any other parenteral route usually employed for this purpose by the skilled worker employing whatever means are commonly used for such purposes, for example, hypodermic needle and syringe.

The physiologically active substances which may be employed in the practice of this invention are those substances which are to be parenterally administered to the patient and include substances which are parenterally administered for either therapeutic or diagnostic purposes. The physiologically active substances which may be employed include those biologically active materials which may be of chemical or biological origin and include such substances as antibiotics, hormones, steroids, allergenic substances, allergen extracts, sera, toxins, anti-toxins, vaccines. Preferably, in the practice of the instant invention it is desired to employ those physiologically active substances which are derived from biological sources for example, antibiotics, allergenic materials and extracts, biological extracts, for example, vaccines, sera, and anti-toxins, for it has been determined that such biologically originated materials are most susceptible to storage instability and degradation. The amounts of the physiologically active substances which may be employed in the practice of this invention are those which are normally administered to the patients for the condition being treated or the purpose required, and will depend on the factors usually determined by the skilled worker to control the amounts of the substance being employed.

The final compositions of this invention may be prepared in any manner considered suitable by the skilled worker practicing the invention. The individual components of the final compositions of this invention may be admixed to form the desired compositions, or in some instances where the physiologically active substance is in a dry state, for example, as a result of lyophilization, the said physiologically active substance may first be reconstituted by the addition of the desired liquid vehicle, for example, water, and the required amounts of epsilon-aminocaproic acid or salt thereof then incorporated, for example, by admixing, to yield the final stable final compositions of this invention. The thus storageably stable final compositions may then be held for future use.

The invention may be further illustrated by the following examples:

Example 1

Grass pollen extract (*Lolium perenne*) (commercially available from HAL Allergenen Laboratorium B.V., Haarlem, Holland) was admixed with 10 ml of pyrogen-free, distilled water. To the resultant solution was added with mixing, *epsilon*-aminocaproic acid until the resultant concentration thereof in the final solution was 0.1 M. The resultant final composition was then divided into individual parenterally administerable doses of 0.05 ml each.

### Example 2

The procedure of Example 1 was followed except that an equivalent amount of house dust mite extract (commercially available from HAL Allergenen Laboratorium B.V.) was substituted for the grass pollen extract, yielding equivalent results.

### Example 3

The procedure of Example 1 may be followed except that equivalent amounts of antibiotics, for example, penicillin G, cephalosporin, tetracycline or oxytetracycline; vaccines, for example, pertussis, typhoid, anti-rabies, or yellow fever vaccine; anti-toxins, for example, tetanus anti-toxin, may be substituted for the grass pollen extract, with similar results.

### Example 4

In vivo tests were performed employing the parenteral compositions of this invention. Grass pollen allergen extract and house dust mite allergen extracts were prepared in accordance with the procedures set forth in Example 1. In addition, as controls, compositions were prepared in accordance with the procedures of Example 1 and 2, except that sodium phosphate was substituted for *epsilon*-aminocaproic acid to a concentration of 0.15 M.

The final parenterally administerable extracts had the following compositions:

A. Grass pollen allergen in pyrogen-free distilled water containing 0.1 M *epsilon*-aminocaproic acid.

B. Grass pollen allergens in pyrogen-free distilled water containing 0.15 M sodium phosphate.

C. House dust mite allergens in pyrogen-free distilled water containing 0.1 M *epsilon*-aminocaproic acid.

D. House dust mite allergens in pyrogen-free distilled water containing 0.15 M sodium phosphate.

The respective final extracts (0.05 ml) were intradermally injected into the test subjects forearms at various sites at premeasured distances from each other. These skin tests were read fifteen minutes after administration. The wheal and flare reactions were delineated on a transparent sheet and the mean square wheal diameters ($d^2wh$) were calculated. The results are set forth in Table 1 and show that there is a significant decrease in the local adverse reactions involved in the administration of the compositions of this invention.

<u>TABLE 1</u>

In addition, the final extracts were also intradermally injected into subjects' forearms at various sites at premeasured distances from each other over an extended period of time after preparation. In the case of Compositions A and B, these tests were made over a period of 211 days after preparation of the compositions; and in the case of Compositions C and D, over 210 days after preparation. Each skin test was read fifteen minutes after the administration thereof. The wheal and flare reactions, which is a measure of the potency of the extract composition's allergenicity, were delineated on a transparent sheet and the mean square wheal diameters ($d^2wh$) were calculated. The results set forth on Tables 2 and 3 below demonstrate that while there is a consistent and persistant degradation in the potency of prior art compositions, the compositions of this invention have little or no potency loss over an extended period of time.

## TABLE 2

## TABLE 3

## Example 5

In vitro experiments were performed to demonstrate the improved storage stability of the compositions of the instant invention. A grass pollen extract (*Lolium perenne*) containing 50,000 NU in 50% glycerol was held at −70°C until diluted from 2000 NU in a series of dilutions down to 31 NU, in the following compositions:

A. Grass pollen with 0.154 M phenolated phosphate, pH = 7.

B. Grass pollen with phenolated phosphate containing 0.1 M *epsilon*-aminocaproic acid.

Allergen coupled cellulose discs were made in accordance with the RAST method (A.B. Pharmacia, Sweden) using a bicarbonate buffered *Lolium perenne* extract. A RAST positive sera was used in RAST inhibition tests which were performed on the subject compositions to determine their activity. The results which demonstrate the reduction in the degradation by freezing and thawing of the instant compositions are set forth in Table 4.

### TABLE 4

#### Relative Activity

| | | | |
|---|---|---|---|
| Extract A | 1.0 | 1.0 | 1.0 |
| Extract B | 1.6 | 1.5 | 1.7 |

The above results demonstrate the relative activity of Extract B compared to Extract A and show that the activity of Extract B is substantially greater than that of Extract A.

## Example 6

The extract compositions prepared in Example 5 and containing 2000 NU of grass pollen allergens were then divided into 5 equal portions. One portion was employed as a control and the other four portions were incubated for 6, 24, 48 hours and 7 days respectively, at 37°C. At the end of each time period the test composition was tested in accordance with the RAST inhibition method to determine the relative potencies thereof. The results, which show that the compositions of this invention are more potent than those of the prior art, are set forth in Table 5 below.

### TABLE 5
#### RAST Potencies After:

| | 0 Hrs. | 6 Hrs. | 1 Day | 2 Days | 7 Days |
|---|---|---|---|---|---|
| Extract A | 1.0 | 0.70 | 0.42 | 0.41 | 0.14 |
| Extract B | 1.0 | 0.94 | 0.65 | 0.52 | 0.22 |

These results demonstrate that the shelf life of Extract B is approximately twice that of Extract A.

## Example 7

The procedure of Example 6 was repeated except that the extracts were stored at a temperature of 4°C for 1, 3 and 6 months. The results obtained are set forth in Table 6.

### TABLE 6
#### RAST Potencies After:

| | 0 Month | 1 Month | 2 Months | 3 Months |
|---|---|---|---|---|
| Extract A | 1.0 | 0.77 | 0.55 | 0.36 |
| Extract B | 1.0 | 0.57 | 0.32 | 0.26 |

The foregoing results demonstrate that the shelf life of Extract B is approximately three times that of Extract A.

Example 8

To several patients, two different dosages of Extracts C and D of Example 4 were administered. The initial dosage had a potency of 5 Noon Units (NU) while the second was increased tenfold to 50 NU. The difference in the skin reactivity as a result of the two administrations was measured in accordance with the method set forth in Example 4. The results set forth in Table 7 clearly show that the use of the compositions of the instant invention maintain a dose dependent reaction in a group of patients.

TABLE 7

In addition to the foregoing, it must be noted that the etiology and root causes of the multitude of individual diseases or conditions included within the generic definition of allergy are not completely known at this time. The generic condition known in medicine as "allergy" connotes an altered reaction of tissues or other systemic parameters in certain persons on exposure to certain agents which, in similar amounts are innocuous to other persons. These altered reactions of the allergic person may be of various types, ranging from a more or less intense skin sensitivity to a severe and sometimes fatal systemic shock reaction.

At the present time it is the consensus of the medical world that the allergic reactions suffered by all susceptible persons are mediated, if not basically caused by various exciting agents, commonly referred to as "allergens" or "antigens". The presence of these allergens cause, in those individuals who are sensitive to the specific allergens involved, the production of specific antibodies or sensitized cells. The interaction of antibodies with allergens by some mechanism not fully appreciated, cause allergic symptoms. Common manifestations of the type of allergic symptoms usually seen in persons suffering a so-called immediate-type "allergic attack" are such conditions as "hay fever" i.e., runny nose and watery eyes, asthmatic reactions, gastrointestinal disturbances, urticaria edema and shock.

A person who is allergic may be sensitive to only one allergen, but multiple sensitivities are the rule. The various types of allergens to which a patient may be susceptible are found in such allergic materials as inhalants, for example pollens, fungi, vegetable or animal epithelia, cosmetics, and house dust components; foods, for example, eggs, shellfish, and strawberries; infectious agents, for example, bacteria, fungi, molds, parasites, such as, mites, ticks or fleas; and contactants, for example, plants, flowers, chemicals, furs, and cosmetics. These allergic materials may contain one or more allergens to which the patient is sensitive and exposure to these allergic materials will, in the susceptible individual, cause an allergic attack, manifested by one or more allergic symptoms, the severity of which depends upon the degree of exposure as well as the nature of the allergic material to which the patient is exposed.

It is important to determine the types of allergic materials and/or allergens to which the allergic person is susceptible. This determination is required in order to know which allergic materials the patient is to be isolated from as well as to ascertain the types of treatment which will be required to prevent, ameliorate or therapeutically treat the patient. The diagnostic procedures that have been developed to date include one that is most commonly employed to determine the identity of the allergens to which the patient is sensitive, the skin test. In the skin test diagnostic procedure a small amount of an extract of the allergen is administered cutaneously to the patient and the local allergic reaction, i.e., wheal and flare, observed to determine the patients sensitivity to the test allergen.

Once the identity of the allergens to which the patient is sensitive has been determined, the type of therapeutic treatment required to prevent future allergic attacks can be decided upon. Where the total elimination of the allergen from the presence of the patient is not feasible, for example, where the allergen is a pollen or house dust component, one of the prime methods of treatment is that of desensitization or hyposensitization. This method of treatment involves the successive injecting of an extract of the causitive allergen in a series of gradually increasing doses until the patient slowly builds up an immunity threshold which will permit exposure to a normal concentration of the allergen without the initiation of an allergic attack.

As noted above, the allergen extracts presently employed have the ability to degrade and lose substantial potency within a short time after their production or reconstitution, if they are stored in the dried state for later use. In addition, desensitization procedures must be carried out with great caution, especially in those cases where the allergen is highly potent or the patient is extremely sensitive, as severe reactions may result. The reactions involved may range from severe local reaction and swelling at the site of injection to severe systemic reaction, including anaphylactic shock and death.

We have now discovered a process of producing allergen containing extracts adapted for systemic administration to patients and which are suitable for use in the diagnosis and therapeutic treatment of allergic conditions while avoiding the above noted disadvantages, which comprises first extracting allergic materials with a solvent having incorporated therein a small but effective amount of epsilon-aminocaproic acid or a non-toxic pharmaceutically acceptable salt thereof.

The allergic materials which may be employed in the practice of this invention may be those materials which contain allergens to which patients are sensitive and which are noted above.

The suitable extraction solvent which may be employed in the practice of the instant invention may be any solvent which will satisfactorily extract the allergens from the allergic material, and/or which will be suitable for systemic administration to the patient being treated, or alternatively is capable of being completely removed from the extracted allergen-containing composition prior to its administration to the patient. Most preferably, it has been found that an aqueous solvent provides the most satisfactory result in the practice of this invention. However, the instant invention may be practiced by employment of any suitable solvent which can be used for the purposes required, including alcohols, such as ethanol; pyridine/aqueous alkaline extraction solutions; and other like solvents which are known to the skilled worker to be useful for such purposes.

The satisfactory practice of the instant invention requires that a small but effective amount of epsilon-aminocaproic acid or a salt thereof be incorporated in the solvent of extraction. By a small but effective amount it is hereby meant that the final concentration of epsilon-aminocaproic acid or a salt thereof in the extraction composition should be between about 0.001 M and 0.5 M, and preferably between 0.025 M and 0.4 M, and most preferably between 0.05 and 0.2 M, although the other concentrations may also provide satisfactory results.

The conditions under which the extraction of the allergic materials may be conducted are those under which such extraction procedures are usually performed, as is well known to the skilled worker. Thus, the extraction procedures may be performed at any temperature or under such atmospheric conditions as may provide the most optimum results as is appreciated by the skilled worker. The allergen containing extract composition obtained by the extraction of the allergic material with the solvent composition of this invention may then be treated in the same manner as any allergen containing extract. Thus, the allergen containing extract may be employed directly for diagnosis of therapeutic treatment of patients having an allergic condition; particularly in the case of diagnostic purposes any local reaction which is not of diagnostic importance will be inhibited; or alternatively, the said allergen containing extract may be further treated to obtain a composition having a higher, although innocuous, content of allergens; or as a further alternative the allergen containing extract may be taken down to dryness by any suitable procedure known to the skilled worker to accomplish this result without injury to the allergens, for example, lyophilization, for storage and reconstitution at a later date just prior to administration to the patient.

The instant invention may be further illustrated by the following examples:

## Example 9

An extraction solution comprised of pyrogen-free distilled water containing 0.1 M of *epsilon*-amino caproic acid, and having a pH of 7.0 was prepared. The resultant extraction solution was then employed to extract the allergens of *Lolium perenne* pollen, which was carried out under normal laboratory conditions of temperature and pressure to yield an allergen-containing extract solution having incorporated therein approximately 0.1 M of *epsilon*-amino caproic acid. To this final allergen containing solution was added a small amount of phenol as a preservative.

The foregoing procedure may be followed except that 0.001 M and 0.5 M of *epsilon*-amino caproic acid is substituted for the 0.1 M employed, with like results being obtained.

The foregoing procedure may be followed except that equivalent amounts of house dust components, mites, molds, ragweed pollen and animal danders may be employed in place of the *Lolium perenne* pollen to obtain like results.

Example 10

In vitro tests to determine the specificity and potency of the allergen-containing extracts of the instant invention were conducted as follows:

An amount of grass pollen (*Lolium perenne*) was extracted with extraction solutions of the following compositions:

1. Pyrogen-free distilled water containing 0.1 M sodium bicarbonate pH = 7.0
2. Pyrogen-free distilled water containing 0.154 M sodium phosphate, pH = 7.0
3. Pyrogen-free distilled water containing 0.1 M epislon aminocaproic acid (EACA), pH = 7.0.

The resultant extracts were then tested on cellulose discs prepared and assayed according to the RAST method (RAST assay method commercially available from AB Pharmacia, Sweden) according to the following general procedure: Fifty micro-liters of inhibiting extract was incubated with 50 microliters of a highly positive grass pollen serum pool. After 3 hours of incubation, *Lolium perenne* allergen-coupled discs were added to the tubes. After 6 hours of incubation, discs were washed 3 times and incubated with 50 microliters of radiolabelled anti IgE for at least 6 hours. After washing, radioactivity was measured in a gamma counter and the % of inhibition was calculated.

RAST values for 6 individual sera were determined with discs coupled to *Lolium perenne* extract produced with Extraction solutions 1, 2 and 3, with discs obtained from AB Pharmacia serving as a control. Results are shown in Table 8.

RAST values were determined for various dilutions of a positive serum pool with discs coupled to *Lolium perenne* extract produced with Extraction solution 1 and 3. Results are shown in Table 9.

Extracts made with Extraction solutions 1, 2 and 3 were assayed by the RAST inhibition method. Results are shown in Table 10.

The results of these tests demonstrate that the compositions of the instant invention have the same potency as the prior art compositions.

TABLE 8

TABLE 9

## EP 0 106 727 B1

### TABLE 10

```
                              •  Extract 1
                              ▫  Extract 2
Inh   %                       o  Extract 3

80

60

40

20

      15  21  27  33  40  log conc. x 10
```

### Claims

1. A composition suitable for parenteral administration, which consists of an allergenic extract in parenterally administerable form having incorporated therein an amount of 0,001—0,5 M epsilon-aminocaproic acid per dosage unit or a corresponding amount of a non-toxic pharmaceutically acceptable salt thereof.

2. The method of improving the storage stability of parenterally administerable allergenic extract compositions according to Claim 1 which comprises incorporating in said compositions an amount of epsilon-aminocaproic acid or a non-toxic pharmaceutically acceptable salt thereof as defined in Claim 1.

3. A method of producing allergen containing extracts adapted for systemic parenteral administration to patients according to Claim 1 and which are suitable for use in the diagnosis or therapeutic treatment of allergic conditions, which comprises first extracting allergic materials with a solvent having incorporated therein an amount of epsilon-aminocaproic acid or a non-toxic, pharmaceutically acceptable salt thereof as defined in Claim 1 and obtaining the allergen containing extract and thereafter the composition thus produced.

4. The method of Claim 3 wherein the solvent is water.

### Patentansprüche

1. Zur parenteralen Verabreichung geeignete Zusammensetzung, die aus einem allergenen Extrakt in parenteral verabreichbarer Form besteht, in den eine Menge von 0,001—0,5 M ε-Aminocapronsäure pro Dosiseinheit oder eine entsprechende Menge eines nicht-toxischen, pharmazeutisch annehmbaren Salzes derselben eingemischt ist.

2. Verfahren zum Verbessern der Lagerfähigkeit von parenteral verabreichbaren allergenen Extraktzusammensetzungen nach Anspruch 1, dadurch gekennzeichnet, daß eine wie in Anspruch 1 definierte Menge von ε-Aminocapronsäure oder eines nicht-toxischen, pharmazeutisch annehmbaren Salzes derselben in besagte Zusammensetzungen eingemischt wird.

3. Verfahren zum Herstellen von Allergene enthaltenden Extrakten, geeignet zur systemischen parenteralen Verabreichung an Patienten, nach Anspruch 1, die zur Verwendung in der Diagnose oder der therapeutischen Behandlung von allergischen Zuständen geeignet sind, dadurch gekennzeichnet, daß zunächst allergische Materialien mit einem Lösungsmittel, in das eine wie in Anspruch 1 definierte Menge von ε-Aminocapronsäure oder eines nicht-toxischen, pharmazeutisch annehmbaren Salzes derselben eingemischt ist, extrahiert und das Allergene enthaltende Extrakt und danach die so hergestellte Zusammensetzung gewonnen werden.

4. Verfahren nach Anspruch 3, dadurch gekennzeichnet, daß das Lösungsmittel Wasser ist.

### Revendications

1. Composition convenant à l'administration parentérale, qui se compose d'un extrait allergénique sous une forme administrable par voie parentérale, à laquelle est incorporée une quantité de 0,001 à 0,5 M d'acide epsilon-aminocaproïque par unité posologique, ou bien une quantité correspondante d'un sel non toxique et pharmaceutiquement acceptable de cet acide.

2. Procédé d'amélioration de la stabilité de conservation de compositions d'extraits allergéniques administrables par voie parentérale selon la revendication 1, qui consiste à incorporer auxdites compositions une quantité d'acide epsilon-aminocaproïque ou d'un sel non toxique et pharmaceutiquement acceptable de celui-ci, telle qu'elle est définie dans la revendication 1.

11

3. Procédé de production d'extraits contenant des allergènes adaptés pour l'administration parentérale systémique à des patients, selon la revendication 1, et qui conviennent à l'utilisation pour le diagnostic ou le traitement thérapeutique d'états allergiques, qui consiste à extraire d'abord des substances allergiques avec un solvant auquel est incorporée une quantité d'acide epsilon-aminocaproïque ou d'un sel non toxique et pharmaceutiquement acceptable de celui-ci, telle qu'elle est définie dans la revendication 1, et à obtenir l'extrait contenant les allergènes, et ensuite la composition ainsi produite.

4. Procédé selon la revendication 3, dans lequel le solvant est l'eau.